(19) Europäisches Patentamt
European Patent Office
Office européen des brevets

(11) **EP 2 829 229 A1**

(12) **EUROPEAN PATENT APPLICATION**
published in accordance with Art. 153(4) EPC

(43) Date of publication:
**28.01.2015 Bulletin 2015/05**

(51) Int Cl.:
***A61B 5/16*** (2006.01)   ***A61B 5/0456*** (2006.01)

(21) Application number: **12872002.6**

(22) Date of filing: **19.03.2012**

(86) International application number:
**PCT/JP2012/057071**

(87) International publication number:
**WO 2013/140523 (26.09.2013 Gazette 2013/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Fujitsu Limited
Kawasaki-shi, Kanagawa 211-8588 (JP)**

(72) Inventors:
• **SANO, Satoshi
Kawasaki-shi
Kanagawa 211-8588 (JP)**

• **MASUDA, Yuta
Kawasaki-shi
Kanagawa 211-8588 (JP)**
• **ODAGIRI, Junichi
Kawasaki-shi
Kanagawa 211-8588 (JP)**

(74) Representative: **Lewin, David Nicholas
Haseltine Lake LLP
Lincoln House, 5th Floor
300 High Holborn
London WC1V 7JH (GB)**

(54) **AWAKING DEGREE DETERMINATION DEVICE, AWAKING DEGREE DETERMINATION PROGRAM, AND AWAKING DEGREE DETERMINATION METHOD**

(57)    An awakening-degree determining device (100) according to the present embodiment acquires heartbeat signal data on the subject from a heartbeat detecting unit and detects heartbeat interval data. The awakening-degree determining device (100) calculates the power spectral density that corresponds to each frequency by using the heartbeat interval data, calculates a peak P and a bottom B, and calculates a state index S. Then, the awakening-degree determining device (100) plots the movement of the state index on an awakening-degree determination graph and determines the degree of awakening of the subject on the basis of the position of the state index S.

FIG.5

**Description**

Technical Field

The present invention relates to an awakening-degree determining device, or the like.

Background Art

As the technique for measuring the sleepiness or the degree of awakening of the subject without imposing loads on the subject, there is a frequency analysis technology that uses a heartbeat signal, or the like, of the subject. For example, there is a conventional technology in which the frequency at the peak of the fluctuation of a heartbeat signal and the power spectral density are used as the feature values and the degree of awakening of the subject is determined on the basis of the movement of the feature values.

Citation List

Patent Literature

Patent Literature 1: International Publication Pamphlet No. WO 2008/065724

Summary

Technical Problem

However, the above-described conventional technology has a problem in that it is difficult to accurately determine the degree of awakening of the subject.
For example, if the subject feels sleepy but struggles against sleepiness, the degree of awakening that is determined on the basis of the feature value in the conventional technology is sometimes different from the actual degree of awakening of the subject.
The present invention has been made in consideration of the foregoing and has an object to provide an awakening-degree determining device, an awakening-degree determination program, and an awakening-degree determination method that make it possible to accurately determine the degree of awakening of the subject.

Solution to Problem

According to an aspect of the embodiment of the invention, an awakening-degree determining device includes a memory and a processor coupled to the memory, wherein the processor executes a process including: calculating a heartbeat interval by using a heartbeat signal of a subject; calculating a power spectral density of each frequency by performing a frequency analysis on the heartbeat interval; extracting a combination of a maximum point of the power spectral density and a frequency that corresponds to the maximum point and a combination of a minimum point of the power spectral density and a frequency that corresponds to the minimum point; and determining a degree of awakening of the subject by using a combination of the maximum point and the frequency that corresponds to the maximum point and the combination of the minimum point and the frequency that corresponds to the minimum point.

Advantageous Effects of Invention

The disclosed awakening-degree determining device produces an advantage such that the degree of awakening of the subject can be accurately determined.

Brief Description of Drawings

FIG. 1 is a functional block diagram that illustrates a configuration of an awakening-degree determining device according to a first embodiment.
FIG. 2 is a graph that illustrates an example of heartbeat signal data.
FIG. 3 is a graph that illustrates an operation of a heartbeat interval calculating unit.
FIG. 4 is a graph that illustrates an example of heartbeat interval variation data.

FIG. 5 is a graph that illustrates the relationship between a frequency and a power spectral density.

FIG. 6 is a graph that illustrates an example of an awakening-degree determination graph.

FIG. 7 is a graph that illustrates an example of a change in power spectral density data.

FIG. 8 is a flowchart that illustrates the steps of an operation of the awakening-degree determining device according to the first embodiment.

FIG. 9 is a graph that illustrates an advantage of the awakening-degree determining device according to the first embodiment.

FIG. 10 is a diagram that illustrates an example of a computer that executes an awakening-degree determination program.

Embodiments for Carrying Out the Invention

A detailed explanation is given below, with reference to the drawings, of an embodiment of an awakening-degree determining device, an awakening-degree determination program, and an awakening-degree determination method according to the present invention. The present invention is not limited to the embodiment.

First Embodiment

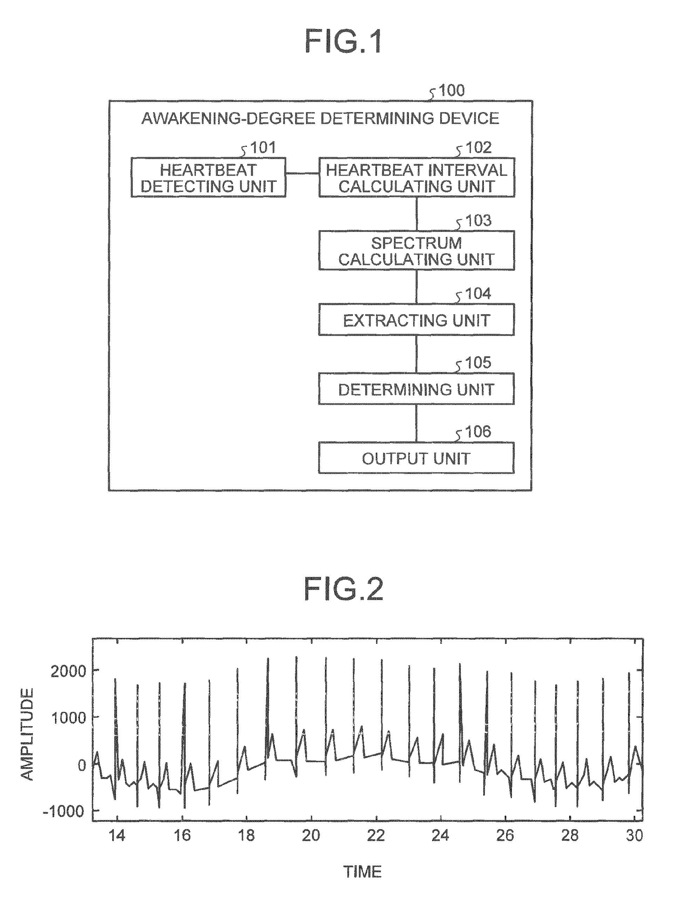

An explanation is given of an awakening-degree determining device according to a first embodiment. FIG. 1 is a functional block diagram that illustrates a configuration of the awakening-degree determining device according to the first embodiment. As illustrated in FIG. 1, an awakening-degree determining device 100 includes a heartbeat detecting unit 101, a heartbeat interval calculating unit 102, a spectrum calculating unit 103, an extracting unit 104, a determining unit 105, and an output unit 106.

The heartbeat detecting unit 101 is a unit that detects a heartbeat signal of the subject. The heartbeat detecting unit 101, for example, acquires a heartbeat signal of the subject by using the potential difference of the electrodes that are in contact with the subject. For instance, the electrodes are provided on the steering wheel of a vehicle, or the like, and, while the subject is driving, a heartbeat signal may be detected from the subject. Furthermore, for example, a pulse signal may be acquired and detected by an ear-clip type photoplethysmographic sensor.

FIG. 2 is a graph that illustrates an example of heartbeat signal data. The horizontal axis of FIG. 2 indicates the time, and the vertical axis indicates the amplitude of a heartbeat signal. As illustrated in FIG. 2, the heartbeat signal data has the amplitude peak that appears with a constant period.

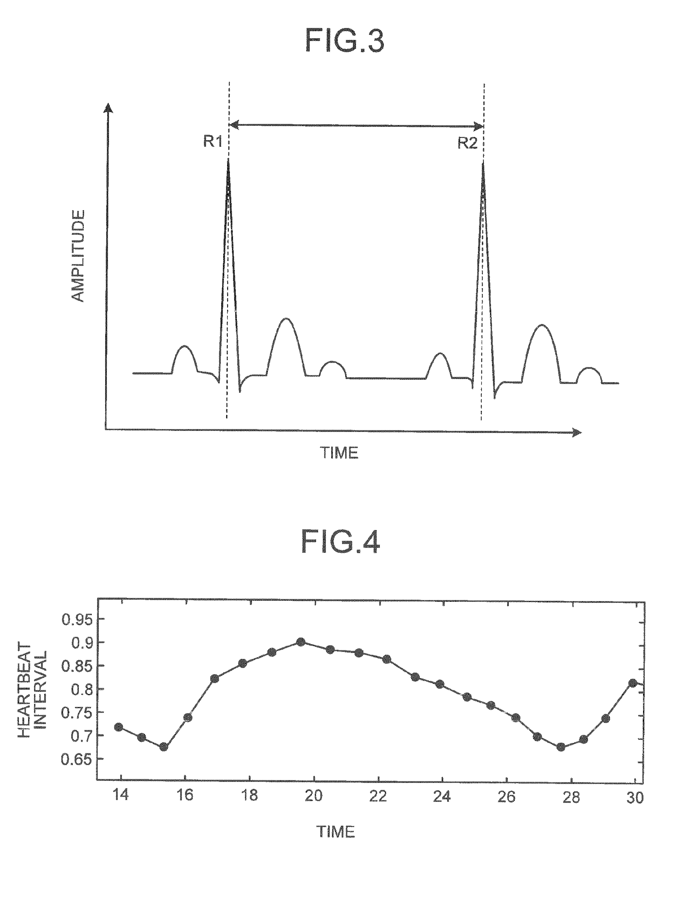

The heartbeat interval calculating unit 102 is a processing unit that detects the timing of each amplitude peak of a heartbeat signal on the basis of the heartbeat signal data and that detects the interval of the timings of the amplitude peaks. FIG. 3 is a graph that illustrates an operation of the heartbeat interval calculating unit. In FIG. 3, the horizontal axis indicates the time, and the vertical axis indicates the amplitude of a heartbeat signal. The signal of FIG. 3 is part of the heartbeat signal data illustrated in FIG. 2.

The heartbeat interval calculating unit 102 detects, as the amplitude peak, the point where the amplitude of a heartbeat signal is equal to or more than a threshold. In the example illustrated in FIG. 3, the amplitude interval calculating unit 102 detects amplitude peaks R1, R2. Then, the heartbeat interval calculating unit 102 detects the time interval between the timing of the amplitude peak R1 and the amplitude peak R2. The time interval between the amplitude peaks is referred to as the heartbeat interval.

The heartbeat interval calculating unit 102 sequentially detects the heartbeat interval and outputs, to the spectrum calculating unit 103, data on the detected heartbeat interval. In the following explanation, data on a heartbeat interval is referred to as heartbeat interval data. Furthermore, the method for detecting the amplitude peak is not limited to the above-described method, and it is possible to use, for example, a method for detecting a peak by performing pattern matching on the basis of an amplitude waveform, a method of using the largest value of the differential coefficient of a pulse signal, or the like.

The spectrum calculating unit 103 is a processing unit that calculates the power spectral density with respect to the variation of the heartbeat interval on the basis of the heartbeat interval data. Here, a detailed explanation is given of an operation of the spectrum calculating unit 103. First, the spectrum calculating unit 103 uses the heartbeat interval data to generate data on a heartbeat interval that varies due to the time passage. Data on a heartbeat interval that varies due to the time passage is referred to as heartbeat interval variation data.

FIG. 4 is a graph that illustrates an example of the heartbeat interval variation data. In FIG. 4, the horizontal axis indicates the time, and the vertical axis indicates the magnitude of a heartbeat interval. As illustrated in FIG. 4, the heartbeat interval varies in accordance with a time change.

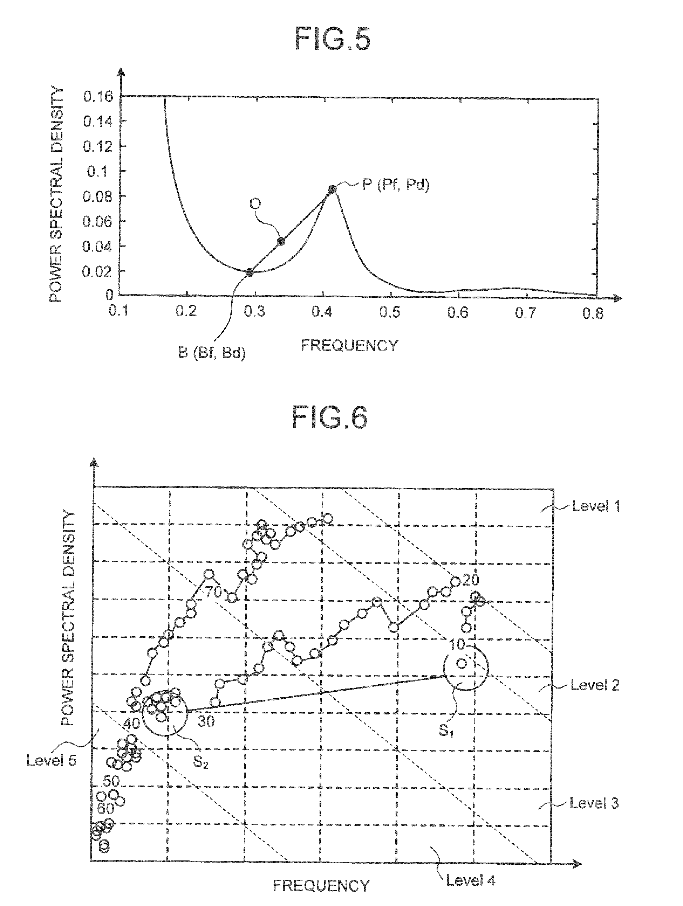

The spectrum calculating unit 103 calculates the power spectral density that corresponds to each frequency on the basis of the heartbeat interval variation data. FIG. 5 is a graph that illustrates the relationship between a frequency and a power spectral density. The horizontal axis of FIG. 5 indicates the frequency, and the vertical axis indicates the magnitude of the power spectral density. The spectrum calculating unit 103 outputs, to the extracting unit 104, data on the power spectral density that corresponds to each frequency. In the following explanation, data on the power spectral density that corresponds to each frequency is referred to as power spectral density data.

Furthermore, the method used by the spectrum calculating unit 103 to calculate the power spectral density data may be any method. For example, the spectrum calculating unit 103 may calculate the power spectral density data by performing Fourier transform.

Furthermore, the spectrum calculating unit 103 is capable of calculating the power spectral density by using, for example, the AR (Autoregressive) model. As disclosed in Non Patent Literature (Shunsuke Sato, Sho Kikkawa, and Toru Kiryu, Introduction to Biosignal Processing, CORONA publishing Co., Ltd.), or the like, the AR model is the model for representing the state at a certain time by using the linear sum of previous time-series data, and it has a feature in that the clear maximum point can be obtained by using a small volume of data compared to Fourier transform.

The p-order AR model of the time series x(s) can be represented by using Equation (1) that uses the AR coefficient a(m) that is a weight to a previous value and the error term e(s). In an ideal state, e(s) that is included in Equation (1) corresponds to white noise.

$$x(s) = \sum_{m=1}^{P} a(m)x(s-m) + e(s) \qquad (1)$$

Power spectral density $P_{AR}(f)$ can be represented by using Equation (2).

$$P_{AR}(f) = \frac{1}{f_s} \frac{\varepsilon_P}{\left| 1 + \sum_{k=1}^{P} \hat{a}_P(k) e^{-2\pi jkf} \frac{1}{f_k} \right|^2} \qquad (2)$$

In Equation (2), p indicates the identification order, $f_s$ indicates the sampling frequency, and $\varepsilon_p$ indicates the identification error. Furthermore, the following mark indicates the k-order AR counting.

$$\hat{a}_P(k) \qquad (3)$$

The spectrum calculating unit 103 may calculate the power spectral density data on the basis of Equation (2) and the heartbeat interval variation data.

The extracting unit 104 uses the power spectral density data to specify the maximum point and the minimum point of the power spectral density. In the following explanation, the maximum point is referred to as a peak, and the minimum point is referred to as a bottom. An operation of the extracting unit 104 is explained by using FIG. 5.

In the example illustrated in FIG. 5, the extracting unit 104 specifies a peak P and a bottom B. The extracting unit 104 represents the peak P by using Pf and Pd. Pf corresponds to the frequency value that is obtained by subtracting the frequency of a reference point O from the frequency of the peak P. Pd corresponds to the value that is obtained by subtracting the power spectral density of the reference point O from the power spectral density of the peak P. Data on Pf, Pd of the peak P is referred to as P (Pf, Pd) as appropriate.

The extracting unit 104 represents the bottom B by using Bf and Bd. Bf corresponds to the value that is obtained by subtracting the frequency of the bottom B from the frequency of the reference point O. Bd corresponds to the value that is obtained by subtracting the power spectral density of the bottom B from the power spectral density of the reference point O. Data on Bf, Bd of the bottom B is referred to as B (Bf, Bd) as appropriate.

The extracting unit 104 outputs P (Pf, Pd) and B (Bf, Bd) to the determining unit 105. Each time the extracting unit 104 acquires the power spectral density data from the spectrum calculating unit 103, it specifies P (Pf, Pd), B (Bf, Bd) and outputs them to the determining unit 105.

Furthermore, the extracting unit 104 may specify the reference point O in any way. For example, the extracting unit 104 specifies, as the reference point O, the middle point of a line segment that connects the bottom B and the peak P. Moreover, the extracting unit 104 may specify, as the reference point O, the center of gravity of the area that includes the bottom B and the peak P.

The determining unit 105 is a processing unit that determines the degree of awakening of the subject on the basis of P (Pf, Pd), B (Bf, Bd). An explanation is given of an operation performed when the determining unit 105 calculates the degree of awakening.

The determining unit 105 uses P (Pf, Pd), B (Bf, Bd) to calculate state index data. The state index data contains a parameter f and a parameter PSD. The determining unit 105 adds Pf and Bf to calculate the parameter f. The determining unit 105 adds Pd and Bf to calculate the parameter PSD. The state index data is referred to as state index S (f, PSD) below as appropriate.

The determining unit 105 determines the degree of awakening of the subject by using the position of the state index S (f, PSD) that is defined by the parameter f and the parameter PSD and that is on an awakening-degree determination graph. FIG. 6 is a graph that illustrates an example of the awakening-degree determination graph. On the awakening-degree determination graph illustrated in FIG. 6, the horizontal axis indicates the frequency, and the vertical axis corresponds to the magnitude of the power spectral density. The awakening-degree determination graph is divided into the areas of Levels 1 to 5. The area of Level 5 is the area that has the lowest degree of awakening of the subject who is sleepy, and the degree of awakening increases in the order of Level 5, 4, 3, 2, and 1. It is assumed that the scale of the awakening-degree determination graph and the areas of Level 1 to 5 are previously set by the determining unit 105.

The determining unit 105 determines the degree of awakening depending on which area of the awakening-degree determination graph includes the state index S (f, PSD). For example, as illustrated in FIG. 6, if the position that corresponds to the state index S (f, PSD) is $S_1$, the determining unit 105 determines that the degree of awakening of the subject is Level 3. If the position that corresponds to the state index S (f, PSD) is $S_2$, the determining unit 105 determines that the degree of awakening of the subject is Level 4.

Furthermore, the determining unit 105 sequentially acquires P (Pf, Pd) and B (Bf, Bd) from the extracting unit 104 and sequentially calculates the state index S (f, PSD). The determining unit 105 may use the moving direction of the state index S (f, PSD) on the awakening-degree determination graph to determine whether the subject is becoming sleepy or the subject is not becoming sleepy. If the state index S (f, PSD) moves from Level 1 toward Level 5 on the awakening-degree determination graph illustrated in FIG. 6, the determining unit 105 determines that the subject is becoming sleepy. Conversely, if the state index S (f, PSD) moves from Level 5 toward Level 1, the determining unit 105 determines that the subject is not becoming sleepy.

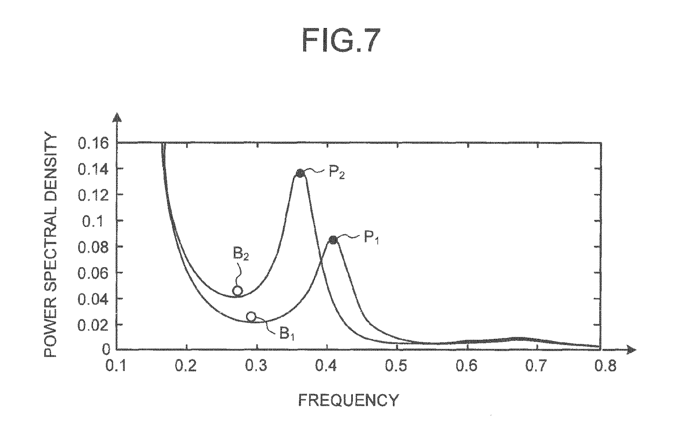

FIG. 7 is a graph that illustrates an example of a change in the power spectral density data. As illustrated in FIG. 7, for example, assume that the power spectral density data is changed, the peak $P_1$ and the bottom $B_1$ are changed to the peak $P_2$ and the bottom $B_2$, and the state index is changed from a state index $S_1$ to a state index $S_2$, as illustrated in FIG. 6. In this case, the state index S (f, PSD) moves from Level 1 toward Level 4. Therefore, the determining unit 105 determines that the subject is becoming sleepy.

The output unit 106 is a processing unit that outputs various types of information in accordance with a determination result of the determining unit 105. For example, the output unit 106 acquires the degree of awakening of the subject from the determining unit 105 and, if the degree of awakening is included in Level 3 to 5, outputs a warning. Furthermore, the output unit 106 may output a warning if the subject is becoming sleepy. Moreover, the output unit 106 may display, on a display device, the position of the state index S (f, PSD) on the awakening-degree determination graph in real time.

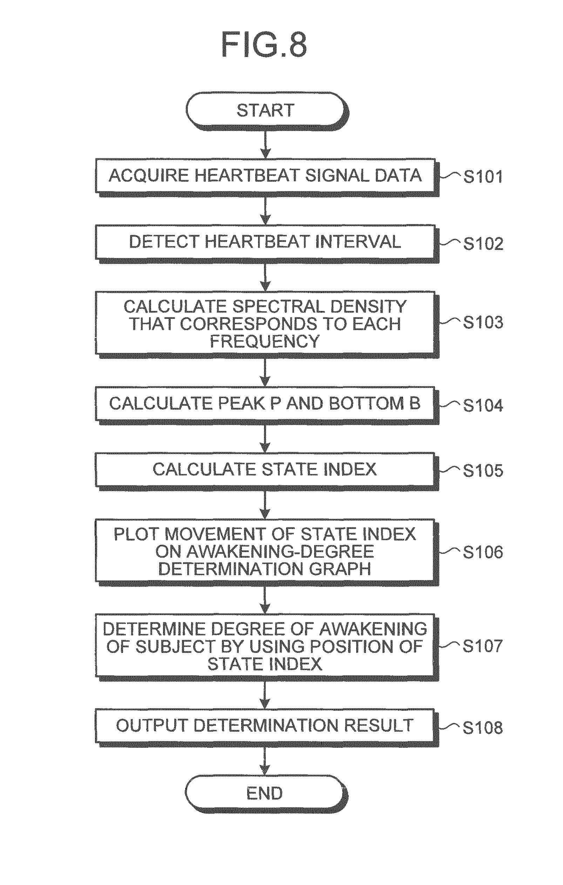

Next, an explanation is given of the steps of an operation of the awakening-degree determining device 100 according to the first embodiment. FIG. 8 is a flowchart that illustrates the steps of an operation of the awakening-degree determining device according to the first embodiment. For example, the operation illustrated in FIG. 8 is performed when the heartbeat detecting unit 101 starts to acquire heartbeat signal data.

As illustrated in FIG. 8, the awakening-degree determining device 100 acquires heartbeat signal data (Step S101) and detects a heartbeat interval (Step S102). The awakening-degree determining device 100 calculates the power spectral density that corresponds to each frequency (Step S103).

The awakening-degree determining device 100 calculates the peak P and the bottom B (Step S104). The awakening-degree determining device 100 uses the peak P and the bottom B to calculate the state index (Step S105).

The awakening-degree determining device 100 plots the movement of the state index on the awakening-degree determination graph (Step S106) and determines the degree of awakening of the subject by using the position of the state index (Step S107). The awakening-degree determining device 100 outputs a determination result (Step S108).

Next, an explanation is given of an advantage of the awakening-degree determining device 100 according to the first embodiment. The awakening-degree determining device 100 performs a frequency analysis on heartbeat signal data to generate power spectral density data in which a frequency and a power spectral density are related to each other. Then, the awakening-degree determining device 100 uses the power spectral density data to specify the peak P and the bottom B and determines the degree of awakening of the subject by using the peak P and the bottom B that are specified. Therefore, with the awakening-degree determining device 100, it is possible to accurately determine the degree of awakening of the subject.

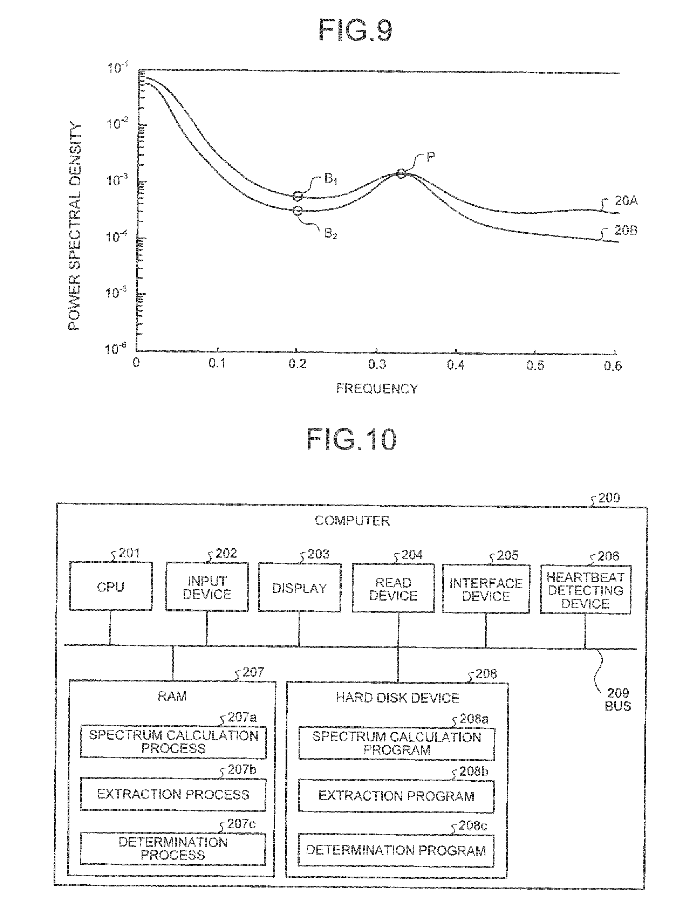

FIG. 9 is a graph that illustrates an advantage of the awakening-degree determining device according to the first embodiment. The horizontal axis of FIG. 9 indicates the frequency, and the vertical axis indicates the power spectral density. FIG. 9 illustrates power spectral density data 20A and power spectral density data 20B. If the degree of awakening decreases, for example, while the subject struggles against sleepiness, the power spectral density data that corresponds to the subject is changed from the power spectral density data 20A to the power spectral density data 20B. It is assumed that the information that the degree of awakening decreases while the subject struggles against sleepiness is, for example, detected by using a method of estimating sleepiness on the basis of facial expressions and the power spectral density data of that time is acquired.

With reference to FIG. 9, if the degree of awakening decreases while the subject struggles against sleepiness, the peak P of each of the power spectral density data 20A, 20B remains the same. Conversely, it is understood that the bottom $B_1$ of the power spectral density data 20B is lower than the bottom $B_2$ of the power spectral density data 20A. The awakening-degree determining device 100 uses not only the peak P but also the bottom B's change to determine the degree of awakening of the subject; therefore, the degree of awakening of the subject can be accurately determined.

Furthermore, the awakening-degree determining device 100 sets the reference point, sets the peak P and the bottom B on the basis of the distance from the reference point, and determines the degree of awakening on the basis of the peak P and the bottom B; therefore, the degree of awakening of the subject can be determined more accurately by using the relative change between the peak P and the bottom B with the reference point as a reference. Furthermore, the awakening-degree determining device 100 sets the reference value each time on the basis of the peak P and the bottom B; therefore, it is possible to dynamically respond to the physical condition of the subject and to determine the degree of awakening accurately even if the physical condition is different from the usual one.

Second Embodiment

Although the embodiment of the present invention has been described heretofore, the present invention may be implemented by using various different configurations other than the above-described first embodiment. An explanation is given below of another embodiment that is included in the present invention as a second embodiment.

(1) With regard to a reference point

In the above-described first embodiment, the extracting unit 104 sets the reference point O by using the peak P and the bottom B; however, this is not a limitation. For example, the extracting unit 104 may previously set the specific reference point O for each subject or may adjust the position of the reference point O depending on a subject. As described above, the reference point O is set for each subject, whereby it is possible to accurately determine the specific degree of awakening of each subject.

Furthermore, the determining unit 105 may determine the degree of awakening of the subject by comparing the reference point O with the peak P and the bottom B. For example, if the peak P has a lower power spectral density or frequency than the reference point O, the determining unit 105 may determine that the degree of awakening of the subject decreases. Furthermore, if the bottom B has a higher power spectral density or frequency than the reference value O, the determining unit 105 may determine that the degree of awakening of the subject decreases.

Furthermore, the determining unit 105 may perform an operation by using, in a combined manner, a determination result 1 of the degree of awakening on the basis of the awakening-degree determination graph and the state index S and a determination result 2 on the basis of the comparison of the reference point O with the peak P and the bottom B. If the degree of awakening has a decreasing tendency according to the determination result 1 and the degree of awakening of the subject decreases according to the determination result 2, the determining unit 105 may determine that there is a "high" possibility that the subject falls asleep and may give a warning in louder sound than that of a usual warning.

Conversely, if the degree of awakening has an increasing tendency according to the determination result 1 but the degree of awakening of the subject decreases according to the determination result 2, it may be determined that the reference point O is improperly set, and the reference point O may be set again. For example, the determining unit 105 may calculate the middle point of the line segment between the peak P and the bottom B again and correct the reference point O.

(2) Predictive detection based on the ratio of the peak P to the bottom B

Pd of the peak P is the index that indicates the state where the rhythm of heartbeat is constant and successive. Furthermore, Pf is the index that is proportional to the amount of activity of the subject. Furthermore, Bd, Bf of the bottom B both indicate the disturbance degree of the rhythm of heartbeat, and it is particularly considered that they are the indexes that indicate the sympathetic activity.

It is considered that unbalance between the above-described sympathetic and the parasympathetic is related to sleepiness and awakening. Therefore, a decrease in the degree of awakening can be previously predicted by focusing on a change in the ratio of the peak P to the bottom B.

The determining unit 105 sequentially calculates the struggle degree F=Pd/Bd. If the amount of change in the struggle degree F is equal to or more than a threshold, the determining unit 105 determines that a decrease in the degree of awakening is predicted. If the determining unit 105 determines that a decrease in the degree of awakening is predicted, it may give a warning to the subject. As the determining unit 105 performs the above operation, it is possible to prevent a decrease in the degree of awakening before it occurs.

(3) Configuration of a system, and the like

Among the operations described in the present embodiment, all or some of the operations that are automatically performed as described above may be performed manually, or all or some of the operations that are manually performed as described above may be performed automatically by using a known method. Furthermore, the operation procedures, the control procedures, the specific names, and the information including various types of data and parameters as described in the above specifications and the drawings may be arbitrarily changed except as otherwise noted.

Furthermore, the components of the awakening-degree determining device 100 that is described in the embodiment are functionally conceptual and do not always need to be physically configured as illustrated in the drawings. Specifically, specific forms of separation and combination of each unit are not limited to those depicted in the drawings, and a configuration may be such that all or some of the units are functionally or physically separated or combined in an arbitrary unit depending on various types of loads or usages. Furthermore, all or any of various processing functions performed by each unit may be implemented by a CPU or a program that is analyzed and executed by the CPU, or it may be implemented as hardware by a wired logic.

FIG. 10 is a diagram that illustrates an example of a computer that executes an awakening-degree determination program. As illustrated in FIG. 10, a computer 200 includes a CPU 201 that performs various calculation operations; an input device 202 that receives an input of data from users; and a display 203. The computer 200 further includes a read device 204 that reads a program, or the like, from a storage medium; and an interface device 205 that communicates data with a different computer via a network. The computer 200 further includes a heartbeat detecting device 206 that detects a heartbeat signal of the subject. The computer 200 further includes a RAM 207 that temporarily stores various types of information; and a hard disk device 208. The devices 201 to 208 are connected to a bus 209.

The hard disk device 208 includes, for example, a spectrum calculation program 208a, an extraction program 208b, and a determination program 208c. The CPU 201 reads the programs 208a to 208c and loads them into the RAM 207.

The spectrum calculation program 208a functions as a spectrum calculation process 207a. The extraction program 208b functions as an extraction process 207b. The determination program 208c functions as a determination process 207c.

For example, the spectrum calculation process 207a corresponds to the spectrum calculating unit 103. The extraction process 207b corresponds to the extracting unit 104. The determination process 207c corresponds to the determining unit 105.

The programs 208a to 208c do not always need to be initially stored in the hard disk device 207. For example, the programs are stored in a "portable physical medium", such as a flexible disk (FD), CD-ROM, DVD disk, magnet-optical disk, or IC card, which is inserted into the computer 200. The computer 300 reads the programs 208a to 208c from the above and executes them.

Reference Signs List

100    AWAKENING-DEGREE DETERMINING DEVICE
101    HEARTBEAT DETECTING UNIT
102    HEARTBEAT INTERVAL CALCULATING UNIT
103    SPECTRUM CALCULATING UNIT
104    EXTRACTING UNIT
105    DETERMINING UNIT
106    OUTPUT UNIT

Claims

1. An awakening-degree determining device comprising:

    a heartbeat interval calculating unit that calculates a heartbeat interval by using a heartbeat signal of a subject;

a spectrum calculating unit that calculates a power spectral density of each frequency by performing a frequency analysis on the heartbeat interval that is calculated by the heartbeat interval calculating unit (102);
an extracting unit that extracts a combination of a maximum point of the power spectral density that is calculated by the spectrum calculating unit and a frequency that corresponds to the maximum point and a combination of a minimum point of the power spectral density and a frequency that corresponds to the minimum point; and
a determining unit that determines a degree of awakening of the subject by using a combination of the maximum point extracted by the extracting unit and the frequency that corresponds to the maximum point and the combination of the minimum point and the frequency that corresponds to the minimum point.

2. The awakening-degree determining device according to claim 1, wherein the determining unit calculates a state index, the state index being a combination of a power spectral density that is obtained by adding the power spectral density of the maximum point and the power spectral density of the minimum point and a frequency that is obtained by adding the frequency that corresponds to the maximum point and the frequency that corresponds to the minimum point, and determines a degree of awakening of the subject by using a change in the state index.

3. The awakening-degree determining device according to claim 2, wherein the extracting unit specifies a reference point by using the maximum point and the minimum point, and the determining unit determines a degree of awakening of the subject by using a change in a distance between the reference point and the maximum point and a change in a distance between the reference point and the minimum point.

4. The awakening-degree determining device according to claim 1, wherein the determining unit determines that the subject is sleepy when the maximum point is lower than the reference point or when the minimum point is higher than the reference point.

5. The awakening-degree determining device according to claim 3 or 4, wherein the extracting unit specifies the reference point for each subject, and the determining unit determines the degree of awakening by using the reference point that corresponds to the subject.

6. The awakening-degree determining device according to claim 1, wherein the determining unit determines whether a decrease in a degree of awakening of the subject is predicted by using a ratio of the power spectral density of the maximum point to the power spectral density of the minimum point.

7. An awakening-degree determination program that causes a computer to execute a process including:

calculating a heartbeat interval by using a heartbeat signal of a subject;
calculating a power spectral density of each frequency by performing a frequency analysis on the heartbeat interval;
extracting a combination of a maximum point of the power spectral density and a frequency that corresponds to the maximum point and a combination of a minimum point of the power spectral density and a frequency that corresponds to the minimum point; and
determining a degree of awakening of the subject by using a combination of the maximum point and the frequency that corresponds to the maximum point and the combination of a minimum point and the frequency that corresponds to the minimum point.

8. An awakening-degree determination method performed by a computer to execute a process including:

calculating a heartbeat interval by using a heartbeat signal of a subject;
calculating a power spectral density of each frequency by performing a frequency analysis on the heartbeat interval;
extracting a combination of a maximum point of the power spectral density and a frequency that corresponds to the maximum point and a combination of a minimum point of the power spectral density and a frequency that corresponds to the minimum point; and
determining a degree of awakening of the subject by using a combination of the maximum point and the frequency that corresponds to the maximum point and the combination of a minimum point and a frequency that corresponds to the minimum point.

# FIG.1

100

AWAKENING-DEGREE DETERMINING DEVICE

101

HEARTBEAT DETECTING UNIT

102

HEARTBEAT INTERVAL CALCULATING UNIT

103

SPECTRUM CALCULATING UNIT

104

EXTRACTING UNIT

105

DETERMINING UNIT

106

OUTPUT UNIT

# FIG.2

# FIG.3

# FIG.4

# FIG.5

# FIG.6

# FIG.7

# FIG.8

START

ACQUIRE HEARTBEAT SIGNAL DATA — S101

DETECT HEARTBEAT INTERVAL — S102

CALCULATE SPECTRAL DENSITY THAT CORRESPONDS TO EACH FREQUENCY — S103

CALCULATE PEAK P AND BOTTOM B — S104

CALCULATE STATE INDEX — S105

PLOT MOVEMENT OF STATE INDEX ON AWAKENING-DEGREE DETERMINATION GRAPH — S106

DETERMINE DEGREE OF AWAKENING OF SUBJECT BY USING POSITION OF STATE INDEX — S107

OUTPUT DETERMINATION RESULT — S108

END

# FIG.9

# FIG.10

<table>
<tr><td colspan="2" align="center"><b>INTERNATIONAL SEARCH REPORT</b></td><td>International application No.<br>PCT/JP2012/057071</td></tr>
</table>

**A. CLASSIFICATION OF SUBJECT MATTER**
*A61B5/16*(2006.01)i, *A61B5/0456*(2006.01)i

According to International Patent Classification (IPC) or to both national classification and IPC

**B. FIELDS SEARCHED**

Minimum documentation searched (classification system followed by classification symbols)
A61B5/16, A61B5/0456

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
WPI

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2008/065724 A1 (Fujitsu Ltd.),<br>05 June 2008 (05.06.2008),<br>paragraphs [0022] to [0040], [0061]<br>& US 2009/0275847 A1 & EP 2087841 A1 | 1,4,5,7,8<br>2,3,6 |
| A | WO 2010/140241 A1 (Fujitsu Ltd.),<br>09 December 2010 (09.12.2010),<br>entire text; all drawings<br>(Family: none) | 1-8 |
| A | JP 2010-131061 A (Toyota Motor Corp.),<br>17 June 2010 (17.06.2010),<br>entire text; all drawings<br>& US 2011/0105925 A1 & WO 2010/001962 A1<br>& CN 102046086 A | 1-8 |

☐ Further documents are listed in the continuation of Box C.    ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search<br>10 May, 2012 (10.05.12) | Date of mailing of the international search report<br>22 May, 2012 (22.05.12) |
|---|---|
| Name and mailing address of the ISA/<br>Japanese Patent Office | Authorized officer |
| Facsimile No. | Telephone No. |

Form PCT/ISA/210 (second sheet) (July 2009)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008065724 A

**Non-patent literature cited in the description**

- **SHUNSUKE SATO ; SHO KIKKAWA ; TORU KIR-YU.** Introduction to Biosignal Processing. CORONA publishing Co., Ltd,